# EUROPEAN PATENT SPECIFICATION

(11) **EP 4 251 123 B1**
(45) Date of publication and mention of the grant of the patent: **16.07.2025**
(21) Application number: 21824666.8
(22) Date of filing: 24.11.2021
(51) Int. Cl.: A61K 8/36, A61K 8/362, A61K 8/365, A61K 8/81, A61K 8/88, A61K 8/897, A61Q 5/06

(54) **COSMETIC COMPOSITION FOR THE TREATMENT OF KERATINIC FIBERS AND METHOD COMPRISING THE APPLICATION OF SAID COMPOSITION TO THE KERATINIC FIBERS**
KOSMETISCHE ZUSAMMENSETZUNG ZUR BEHANDLUNG VON KERATINISCHEN FASERN UND VERFAHREN ZUR BEHANDLUNG VON KERATINISCHEN FASERN
COMPOSITION COSMÉTIQUE POUR LE TRAITEMENT DES FIBRES KERATINIQUES ET PROCÉDÉ COMPRENANT L'APPLICATION DE LADITE COMPOSITION SUR LES FIBRES KERATIQUES

(30) Priority: 30.11.2020 IT 202000028970
(43) Date of publication of application: 04.10.2023
(73) Proprietor: Jean Paul Myne' Srl, 62100 Macerata (MC) (IT)
(72) Inventor: MANNOZZI, Alderano, 63100 Ascoli Piceno (AP) (IT)
(74) Representative: Primiceri, Maria Vittoria
(86) International application number: PCT/IB2021/060915
(87) International publication number: WO 2022/112961

(56) References cited:
- EP-A1- 2 883 535
- EP-A1- 3 228 300
- EP-A1- 3 659 577
- WO-A1-2007/135299
- WO-A1-2012/105985
- WO-A1-2014/072645
- WO-A1-2019/021875

## Description

### Technical field

The present invention relates to cosmetic compositions for the treatment of keratin fibers and to the method for their application on a keratin substrate. In particular, the keratin fibers and the keratin substrate are made up of human hair.

The compositions of the invention are prepared in a cosmetically acceptable medium and the cosmetic treatment method of the keratin substrate provides for the application step on said keratin substrate of a composition as defined below. By means of a single application of at least one of the compositions of the invention, placed in contact with frizzy, curly or wavy keratin fibers (such as hair), it is possible to obtain smooth keratin fibers, with reduced water absorption capacity and greater tensile strength compared to untreated fibers.

### Prior art

It is known that the hair of the human species is composed of a mass of microfibrils composed in turn of amino acidic chains linked together in a different way according to the genetic heritage possessed by the individual.

This category of proteins has a high content of sulfur amino acids (especially cystine and cysteine) as well as significant quantities of acidic amino acids (especially aspartic acid and glutamic acid) and basic amino acids (especially lysine, histidine and arginine).

In \WO2019179745 the chemical-physical characteristics of both the "natural" keratin fibers and those that have suffered damage from the application of cosmetic treatments known to the current state of the art (for instance: bleaching, permanent waving, dyeing, straightening or chemical stretching. (bleaching, waving, dyeing, straightening or stretching) are well described.

The fibers subjected to this type of treatment tend to take on a dull, frizzy appearance over time with poor tensile strength (they break easily).

An improvement in hair straightening procedures is described in patents EP2538916 and EP2595705 which relate to a method for modifying the appearance of keratin fibers through the use of particular operating conditions in the presence of glyoxylic acid to obtain, in a semi-permanent way, the modification of said keratin fibers by transforming them from curved or curly into smooth fibers.

Still in the context of improving the appearance of hair, document WO2011156311 describes the use of compositions based on phenolic compounds and polyamines to allow the protection of a keratin substrate from high energy (UV) sun rays. The composition can be applied indifferently on the hair and on the skin.

US2004191200 discloses cosmetic compositions comprising a thickener in combination with fluorinated polymers in order to coat the keratin fibers of the eyelashes increasing their volume, with a lengthening and separating effect. The composition is applied as it is on the eyelashes and eyebrows.

WO2016033591 describes a hair smoothing composition essentially consisting of at least one derivative of glyoxylic acid formed by the reaction of glyoxylic acid with at least one alpha, beta or gamma amino acid.

US5344903 claims a method of producing copolymers based on perfluoroalkylethyl (meta) acrylates polymerized with alkyl (meta) acrylates for the manufacture of copolymers intended to be used as waterproofing against water and/or oils in natural or synthetic fabrics such as: cotton, cellulose, wool, silk, polyamide as well as on paper and leather.

The need is now felt to improve the procedures for applying glyoxylic acid and make the hair smoothing process faster and more efficient, as well as less stressful for the hair, compared to what is described in EP2538916 and EP2595705. In this patent is in fact described that the hair on which the glyoxylic acid is applied is dried and subjected to straightening at a temperature of 200 ± 50°C without prior rinsing aimed at reducing the amount of substances deposited on the hair during the application of the compositions object of that invention.

Also known (patent application WO2014072645) is a method of fast smoothing/straightening of the hair in which an aqueous cosmetic composition is applied to the hair comprising glyoxylic acid, lactic acid, an amino-modified polyether-silicone copolymer (INCI name: PEG-40/PPG-8 Methylaminopropyl/Hydroxypropyl Dimethicone Copolymer). The cosmetic composition is applied to the keratin fibers for 20 minutes; after which, without performing a rinsing process, a second composition based on fatty alcohols and cationic conditioning agents is applied for 10 minutes; and finally rinse and dry the hair, which is then smoothed with the plate.

However, this method does not allow to persistently smooth/stretch and/or reduce the volume of the hair as the composition applied is not able to modify the keratin fibers and is completely washed away after the first wash. These adverse effects were tested in the comparison tests enclosed to this description in the "Examples" section.

Therefore, the present invention aims to develop a composition that allows to obtain a rapid smoothing/stretching of the hair, associated with a reduction in the volume of the hair, effects that are obtained in an efficient and persistent way while limiting the degradation of the hair. Surprisingly, it has been possible to verify that the smoothing/stretching effects persist even after numerous washings without the need for repeated applications of the composition after each wash.

### Summary of the invention

It has now been surprisingly found that the hair on which glyoxylic acid has been applied can be rinsed to remove excess acid and then directly straightened. This allows the hair to return to a pH similar to the physiological one, before straightening at high temperature, thus minimizing stress on the hair.

This simplification is obtained with the composition of the invention which comprises the combination of glyoxylic acid, or other carboxylic acid of similar properties, with an alkoxylated polyamine and with a fluorinated polymer, as described below.

The invention therefore relates to a composition comprising:
a) a saturated or unsaturated carboxylic acid, and/or its organic and inorganic salts and reaction products between amino acids and carboxylic acids, with carbon number lower than 12, or mixtures thereof;
b) a fluorinated polymer selected from perfluoro-acrylic or perfluoro-silicone polymers, or mixtures thereof;
c) a polyamine substance selected from alkoxylated polyamines or mixtures thereof.

The composition of the invention may further comprise one or more of the ingredients commonly used in the cosmetic industry.

The substances commonly used in the manufacture of cosmetic products are to be understood as those listed in the International Cosmetic Ingredients Dictionary and Handbook, published periodically by the American "Personal Care Products Council" 1620 L Street, NW Washington, D.C.).

These are classified into categories comprising, but not limited to, the following categories: anti-caking agent, antifoam, antimicrobial, antioxidant, antistatic, astringent, binder, whitening, buffering, swelling, chelating, cleansing, cosmetic dye, denaturing, deodorant, detangling, emollient, emulsifier, emulsion stabilizer, film-forming, flavoring, foaming, gelling, hair conditioning, hair fixative, hair waving or smoothing, moistening, hydrotropic, masking, moisturizing, oxidizing, pearlescent, perfuming, plasticizing, preservative, propellant, reducing, re-greasing, refreshing, soluble smoothing, soothing stabilizer, surfactant, tonic, UV filter, viscosity control substances.

The pH of the composition is between 0.5 and 10.0.

The invention also relates to the method of application of said composition on the keratin fibers of the human species.

Further purposes and advantages will become evident from the following detailed description of the invention.

### Detailed description

In the context of this description, the terms "caucasian" type hair and "european" type hair are to be considered synonyms.

According to a preferred - but not limiting - embodiment, the present invention relates to a mixture of organic substances, dispersed in a cosmetically acceptable medium, and at least one method of applying the same mixture to the keratin fibers.

The composition according to the invention comprises:
(a) at least one carboxylic acid having a number of carbon atoms lower than 12 selected from saturated and unsaturated aliphatic carboxylic acids, their salts and mixtures thereof;
(b) at least one fluorinated polymer selected from perfluoro-acrylic or perfluoro-silicone polymers, and mixtures thereof ;
(c) at least one alkoxylated polyamine and mixtures thereof; (d) at least one component selected from water; the composition having a pH between 0.5 and 10.0.

The composition further comprises one or more of the ingredients commonly used in the cosmetic industry.

The composition of the invention is an aqueous composition with a pH between 0.5 and 10.0.

The concentration of each ingredient (a), (b) or (c) is in the range 0.01-30.0% with respect to the overall composition, the rest being water or an aqueous solution applicable on the hair and containing one or more cosmetically acceptable components.

The composition can be used as such on the hair as an aqueous solution or it can be formulated as a cream or gel according to the knowledge of the cosmetic technique, as for instance described in WO2018195614.

The individual components of the composition are detailed as follows:
a) Saturated or unsaturated aliphatic carboxylic acids, and salts thereof having a number of carbon atoms lower than 12 such as: adipic acid, citric acid, tartaric acid, malonic acid, glyoxylic acid dihydroxyacetic acid, fumaric acid, itaconic acid, maleic acid, lactic acid, malic acid, glutaric acid, succinic acid, glycolic acid, and mixtures thereof.
b) Fluorinated polymers representable by the following structures:
   b.1) Silicones with "pendant" perfluorinated chain:
   - Fluorosilicones insoluble in water with molecular weight between 500 and 200,000 daltons of general formula (W): 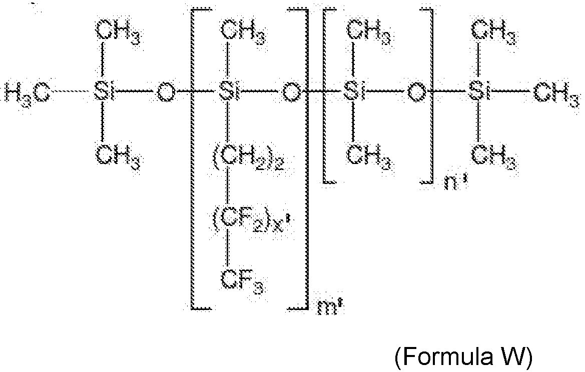 wherein:
      x'= integer between 1 and 50,
      m'= integer between 10 and 1000,
      n'= integer between 10 and 1000.
   - Water-soluble fluorosilicones with the following structural formula and with a molecular weight between 500 and 200,000 daltons: 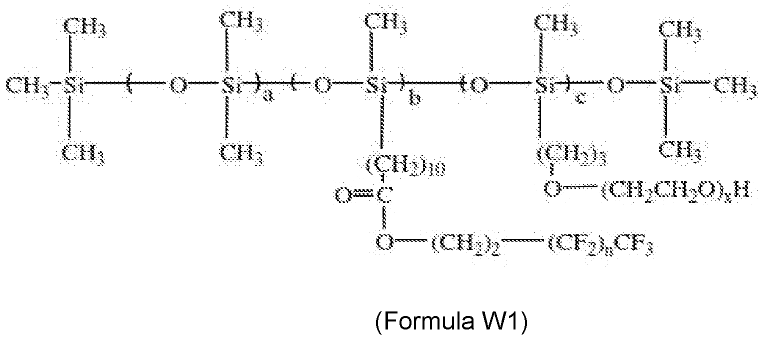 wherein:
      a = integer between 10 and 1000,
      b = integer between 10 and 1000,
      c = integer between 10 and 1000,
      n = integer between 1 and 100,
      x = integer between 10 and 100

The following fluorosilicones belong to these families:
- Perfluorononyl Dimethicone (Cas No 259725-95-6) and
- Perfluorononylethyl Carboxydecyl PEG-10 Dimethicone (CAS n. 500218-22-4) produced by Phoenix Chemical, Inc. - NJ - USA
b.2) - Acrylic and/or methacrylic polymers with non-fluorinated backbone and pendant perfluorinated alkyl groups selected from:
- Diethylaminoethyl methacrylate/hydroxyethyl methacrylate/perfluorohexylethyl methacrylate crosspolymer (produced by Hangzhou Utanpharma biology co. Ltd - China)
- C₆₋₁₄ Perfluoroalkylethyl Acrylate/Hydroxyethyl methacrylate/perfluorohexylethyl methacrylate crosspolymer (produced by Hangzhou Utanpharma biology co. Ltd - China)
- C₁₂₋₁₆ Alkyl PEG-7 Methacrylate/Perfluorohexylethyl Methacrylate copolymer (produced by Biochim srl Milan - Italy)
- Acrylates/Perfluorohexylethyl Methacrylate Copolymer (CAS n. 1557087-30-5) (produced by SEITZ Gmbh - Germany)
- PEG-10 acrylate/perfluorohexylethyl acrylate copolymer (FDA - USA UNII D76Z87928N Rn 910476-54-9)
and mixtures thereof.

Substances described for their chemical, physical and toxicological properties in the "Safety Assessment on Polyfluorinated Polymers as Used in cosmetics - Final Report" Cosmetic Ingredient Review USA - October 16, 2018 and listed in the european database https://ec.europa.eu/growth/tools-databases/cosing/ of substances for cosmetic use and in Commission Decision (EU) 2019/701 of 5 April 2019 (Official Journal of the european community of 08/05/2019).

(c) Alkoxylated polyamines.

Preferred non-limiting examples of suitable alkoxylated polyamines comprise amino molecules, which have at least two primary nitrogen atoms, meaning by this term that at least two primary amino functional groups are present in each molecule, and comprise, but are not limited to, substances corresponding to the following standard formulas:
c.1) - Polyetheramines composed of oxyethylene units (C₂) 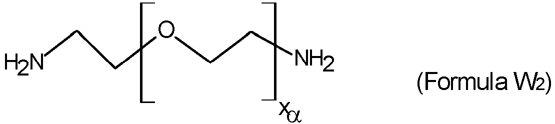 Where Xα is an integer comprised between 1 and 50
c.2) - Polyetheramines composed of oxypropylene units (C₃) 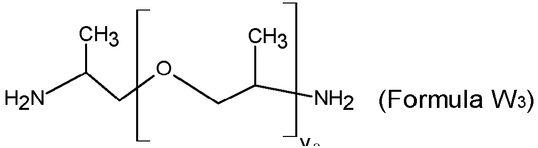 Where yβ is an integer comprised between 1 and 50
c.3) - Polyetheramines composed of mixtures of oxypropylene (C₃) and oxyethylene (C₂) units 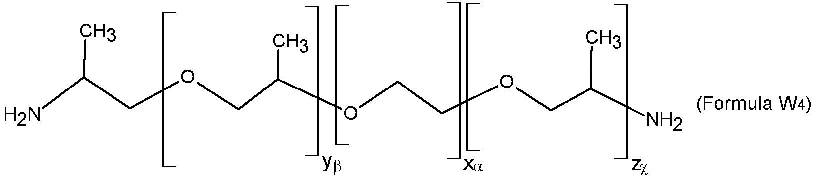 Where xα, yβ and zχ are integers comprised between 1 and 50
c.4) - Polyetheramines composed of tetrahydrofuran units (C₄) 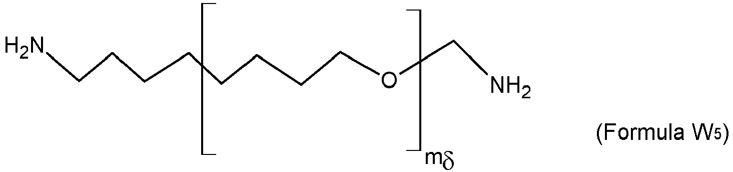 Where mδ is an integer comprised between 1 and 50

Examples of the alkoxylated polyamines for use in the present invention comprise, for instance, diamine compounds belonging to the Jeffamine series, such as the Jeffamine^{®} D and Jeffamine^{®} ED series, JEFFAMINE EDR148 and JEFFAMINE EDR, JEFFAMINE D230, JEFFAMINE D400, JEFFAMINE D2000, JEFFAMINE D4000, JEFFAMINE HK-511, JEFFAMINE ED600, JEFFAMINE ED900 and JEFFAMINE ED2003 available from Huntsman Corporation, Salt Lake City, Utah USA.
c.5) - alkoxylated polyamines corresponding to the formula (W6):

NH₂(CH₂)ₓ∘(OCH₂CH₂O)_{y}∘(CH₂)_{z}∘NH₂ Formula (W6)

where x° and z° are integers between 0 and 3 and y° is an integer between 2 and 100.

Examples of substances belonging to these families are the following:
- PPG-70 bis-(2-aminopropyl) ether (CAS no. 9046-10-0), Poly (propylene glycol) bis (2-aminopropyl ether) - MERCK),
- Bis-aminopropyl diglycol (Cas no. 4246-51-9) (BAXXODUR EC 130 - BASF).

The composition of the invention is a ready-to-use mixture comprising substances (a), (b) and (c) dissolved or dispersed in a cosmetically acceptable aqueous medium having a pH between 0.5 and 10.0.

The concentration of each ingredient (a), (b) or (c) is in the range 0.01-30.0% with respect to the overall composition, the rest being water or a cosmetically acceptable medium (e.g. cream or gel) applicable on the hair.

The term "cosmetically acceptable medium" means the set of substances of common use in the cosmetic art listed in the International Cosmetic Ingredient Dictionary and Handbook published periodically by the American "Personal Care Products Council" 1620 L Street, NW Washington, DC), classified as: anti-caking agent, anti-dandruff, anti-foam, antimicrobial, antioxidant, antistatic, astringent, binder, whitening, buffering, swelling, chelating, cleansing, cosmetic dyeing, denaturing, detangling, emollient, emulsifying, emulsion stabilizer, film-forming, flavoring, foaming, gelling, conditioning, fixative, hair waving or smoothing, moistening, hydrotropic, masking, moisturizing, mattifying, oxidizing, pearlescent, perfuming, plasticizing, solvent, preservative, smoothing, stabilizing, soothing, surfactant, UV filter, viscosity control substances.

The compositions of the invention can be formulated in liquid or semi-liquid, fluid or creamy form, such as: solutions, suspensions, lotions, creams.

The compositions of the invention are applied to the hair with a method that comprises the following basic steps:
I. Applying an amount of the composition of the invention to clean hair (dry or damp);
II. Maintaining said composition in contact with the hair for 5 to 120 minutes;
III. Rinsing the hair and dehumidifying it, drying it with hot air, for instance with a hairdryer,
IV. Straightening the hair with a hair straightener at a temperature of about 200 ± 50°C;

The process of the invention has shown that the keratin fibers after treatment are smooth, even after repeated washes (more than 6), tend to absorb less water in washes and tend to have greater resistance to breakage of the fibers themselves than hair treated with techniques or mixtures commonly used in the cosmetic art. Step (III), which involves the elimination of the excess amount of product compared to that which the single hair can absorb, by means of rinsing with water has proved to be very important in terms of performance as it allows to have a hair at the end of the treatment less damaged (stressed) with consequent improvement of both its appearance and its physical-mechanical characteristics (the hair is more resistant to breakage).

Some experiments were performed to verify the better performance of the mixture object of the invention compared to mixtures already known in the state of the art. In particular, comparisons were made between the mixtures having the best performances described in the patent documents EP2595705 and WO2014072645 and the mixtures object of the present invention with the aim of highlighting the surprising effects achieved with this composition.

The following examples are provided to illustrate the invention.

### APPLICATION EXAMPLES:

Tests were carried out on some of the mixtures of substances belonging to the families of organic substances described in points (a), (b), (c), always used in mixture with each other and dispersed or dissolved in a cosmetically acceptable medium by comparing them with similar mixtures of substances covered by patent documents EP2595705 and WO2014072645. The various components used in the comparative tests are illustrated below:
(a) Carboxylic acid
(b) Fluorinated polymer
(c) Polyamine
(d) Cosmetically acceptable components
(x) mixture of substances containing the copolymer known under the trade name of "Silsoft A+" (INCI Name: PEG-40/ PPG-8 Methylaminopropyl/Hydroxypropyl Dimethicone Copolymer) described and exemplified in WO2014072645

The compositions of the invention were compared with the best compositions based on glyoxylic acid dissolved in a cosmetically acceptable medium as described in EP2595705 with the aim of comparing the results obtainable from the composition object of the present invention with respect to the "state of the art" in hair smoothing described in patent EP2595705.

For all the tests and in all the tables the evaluations were conducted according to the following scheme:

| *(*)* = *Measurement criterion* of *curves reduction* | *No effect* | *Reduction less than 25%* | *Reduction between 26 and 50%* | *Reduction between 51 and* 75% | *Reduction between 76 and 90%* | *Reduction > 90%* |
|---|---|---|---|---|---|---|
| *% Reduction in the number* of *curves found on 30 cm of hair (no. residual curves*/*no. initial curves x 100)* | - | +/- | + | ++ | +++ | ++++ |
| | | | | | | |

| *(**)* = *Measurement criterion of the waterproofing of fibers* | *No effect* | *Reduction less than 5%* | *Reduction between 6 and 15%* | *Reduction between 16 and 30%* | *Reduction between 31 and 50%* | *Reduction > 50%* |
|---|---|---|---|---|---|---|
| *% Reduction* of *fiber drying time on lengths of about 30 cm (no. residual curves*/*no. Initial curves x 100)* | - | +/- | + | ++ | +++ | ++++ |
| | | | | | | |

| *(***) = Criterion for increasing the tensile strength of the hair* | *No effect* | *Increase less than 5%* | *Increase between 6 and* 15% | *Increase between 16 and 30%* | *Increase between 31 and 50%* | Increase > *50%* |
|---|---|---|---|---|---|---|
| *% increase in tensile strength compared to hair (milliJoule) treated with* EP2595705 | - | +/- | + | ++ | +++ | ++++ |

### Example 1:

Comparison of the reduction of the curly aspect, of the waterproofing effect and of the tensile strength of "afro" and "caucasian" (also known as "european") type hair, obtainable on the same using the different types of mixtures object of the present invention, with respect to mixtures covered by patent EP2595705.

The compositions listed in Tables 1A and 1B were prepared: both tables show the details of the results obtained in curves reduction (smoothing) with mixtures containing glyoxylic acid, PPG-70 bis-(2-aminopropyl) ether, bis-aminopropyl diglycol, C₁₂₋₁₆ alkyl PEG-7 methacrylate/perfluoro-hexylethyl methacrylate copolymer and perfluorononylethyl carboxydecyl PEG-10 dimethicone on hair type "AFRO" NATURAL CURLY (Tab. 1A) and on hair of "CAUCASIAN" (also called "european") NATURAL CURLY type (Tab. 1B).

The mixtures were applied to clean and dry hair with an exposure time of about 60 minutes.

Subsequently, the hair treated with the mixtures of the present invention were rinsed with warm water for about 3 minutes in order to eliminate the excess of substances present in the composition.

The hair treated with the invention object of the patent EP2595705 (as described in tables 1A and 1B) have not been rinsed with water.

Then all hair was dried with a hairdryer at a temperature of about 60°C until it was dried according to common use in cosmetics.

Once all the hair samples had dried, they were subjected to plating with a special tool in common use ("flat iron") at a temperature of about 210°C.

Then the hair was subjected to six shampoos applying the common way of use (shampoo application, contact for 5 minutes, rinsing with warm water for 5 minutes, blow-drying) and evaluation of the results as described in table 1.

Tables 1A and 1B show that the advantages that the mixtures of the present invention allow to bring to the current technique in the field of smoothing of keratin fibers with respect to the mixtures described in EP 2595705 are evident.

The mixtures described in EP 2595705, when used according to the method described there, allow to obtain only the smoothing of the hair.

On the contrary, the mixtures of the present invention allow to obtain, in addition to smoothing, a significant reduction in the quantity of water that the hair can absorb and a greater tensile strenght.

Basically, the hair, with a single application becomes: smooth, waterproof and more resistant to breaking by traction.

Furthermore, the tests show that optimal results are achieved only with the combination of the three components (a), (b) and (c), otherwise the tests are not satisfactory.

### Example 2:

Comparison of the reduction of the frizzy appearance, of the waterproofing effect and of the tensile strength of "afro" and "caucasian" (also known as "european") type hair, obtainable on the same using the different types of mixtures object of the present invention, with respect to mixtures covered by patent EP2595705.

The compositions listed in Tables 2A and 2B were prepared. Both tables show the details of the results obtained in volume reduction (frizz) with mixtures containing maleic acid, bis-aminopropyl diglycol and acrylates/perfluorohexylethyl methacrylate copolymer.

Tables 2A and 2B show how the advantages that the mixtures of the present invention allow to bring to the current technique in terms of reducing the frizz effect of keratin fibers are evident compared to the mixtures described in EP 2595705.

Furthermore, the tests show that optimal results are achieved only with the combination of the three components (a), (b) and (c), otherwise the tests are not satisfactory.

### Example 3:

Comparison of the reduction of the curly aspect, of the waterproofing effect and of the tensile strength of "afro" and "caucasian" (also known as "european") type hair, obtainable on the same using the different types of mixtures object of the present invention, with respect to mixtures covered by patent EP2595705.

The compositions listed in Tables 3A and 3B were prepared. Both tables show the details of the results obtained in curves reduction (smoothing).

Tables 3A and 3B show how the advantages that the mixtures of the present invention allow to bring to the current technique in terms of reducing the frizz effect of keratin fibers are evident compared to the mixtures described in EP 2595705.

Furthermore, the tests show that optimal results are achieved only with the combination of the three components (a), (b) and (c), otherwise the tests are not satisfactory.

### Example 4:

Comparison of the reduction of the frizzy appearance, of the waterproofing effect and of the tensile strength of "afro" and "caucasian" (also known as "european") type hair, obtainable on the same using the different types of mixtures object of the present invention, with respect to mixtures covered by patent EP2595705.

The compositions listed in Tables 4A and 4B were prepared. Both tables show the details of the results obtained in volume reduction (frizz).

Tables 4A and 4B show how the advantages that the mixtures of the present invention allow to bring to the current technique in terms of reducing the frizz effect of keratin fibers are evident compared to the mixtures described in EP 2595705.

Furthermore, the tests show that optimal results are achieved only with the combination of the three components (a), (b) and (c), otherwise the tests are not satisfactory.

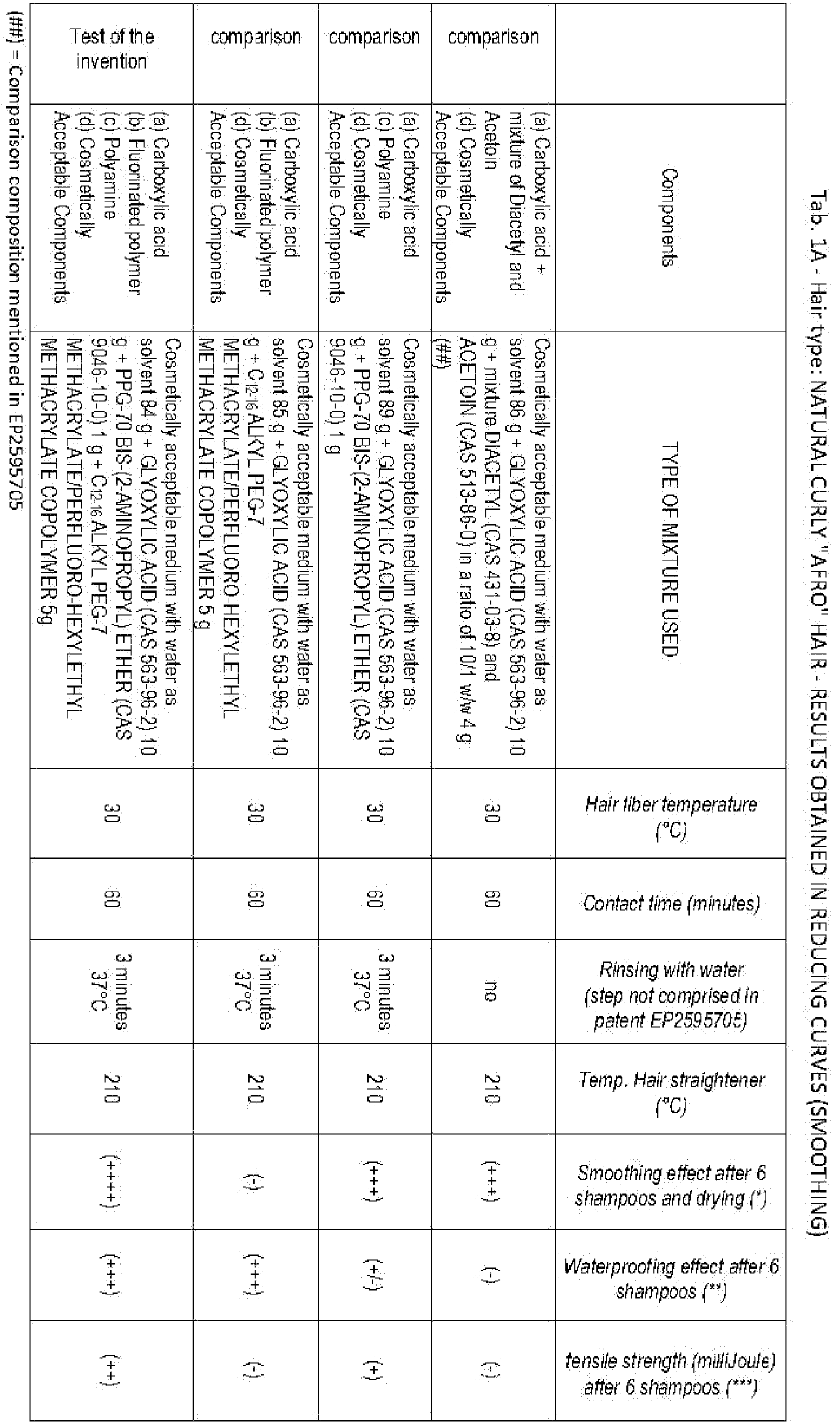

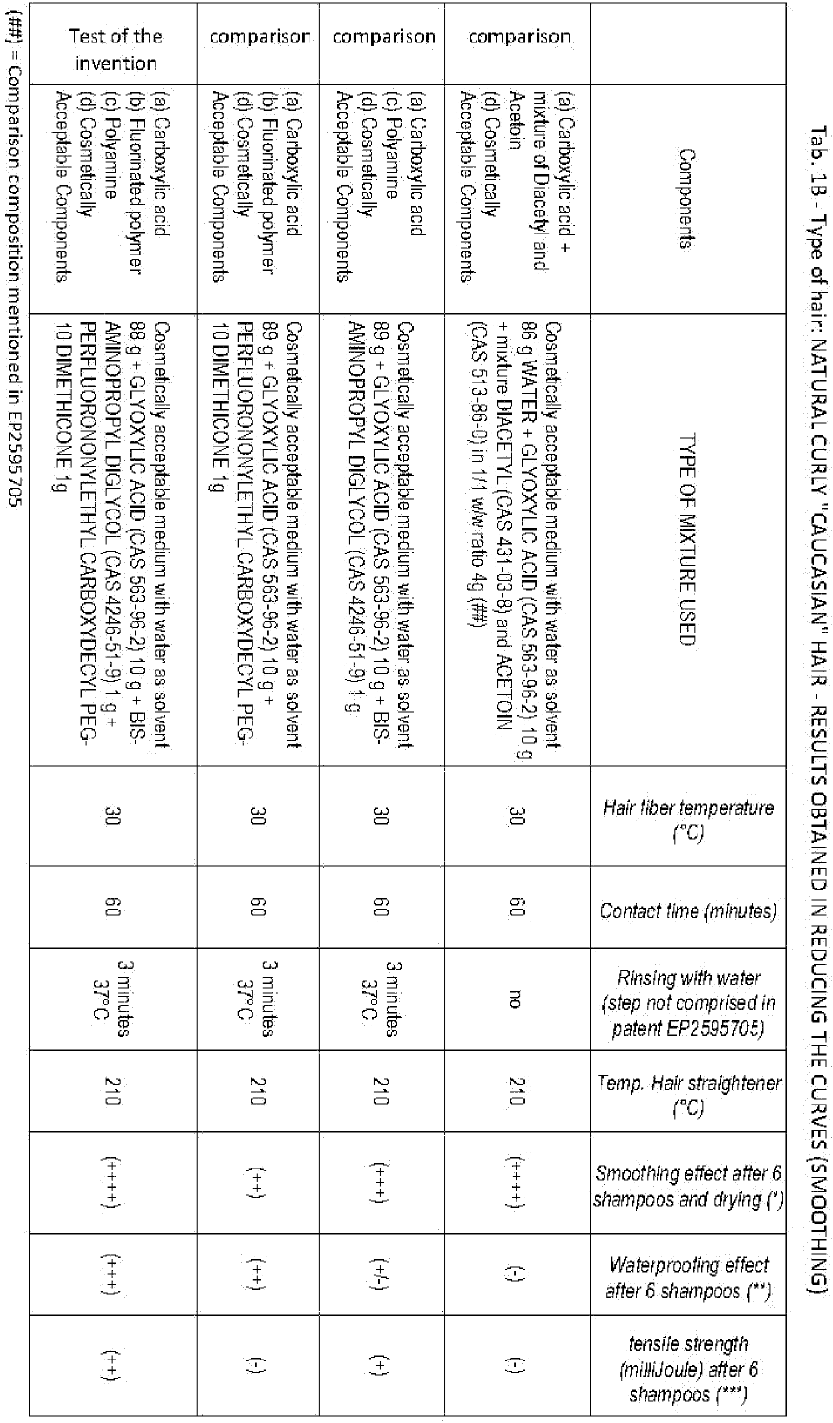

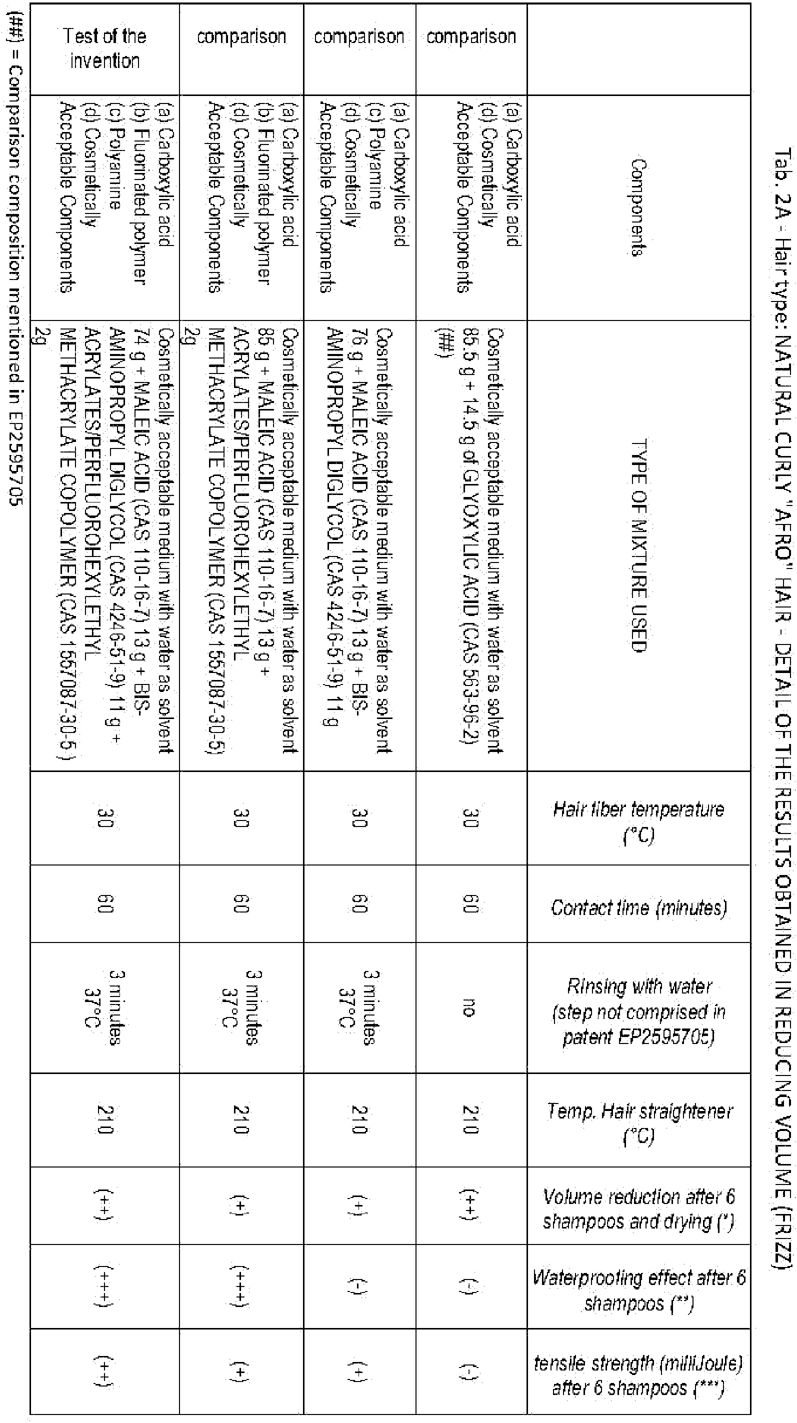

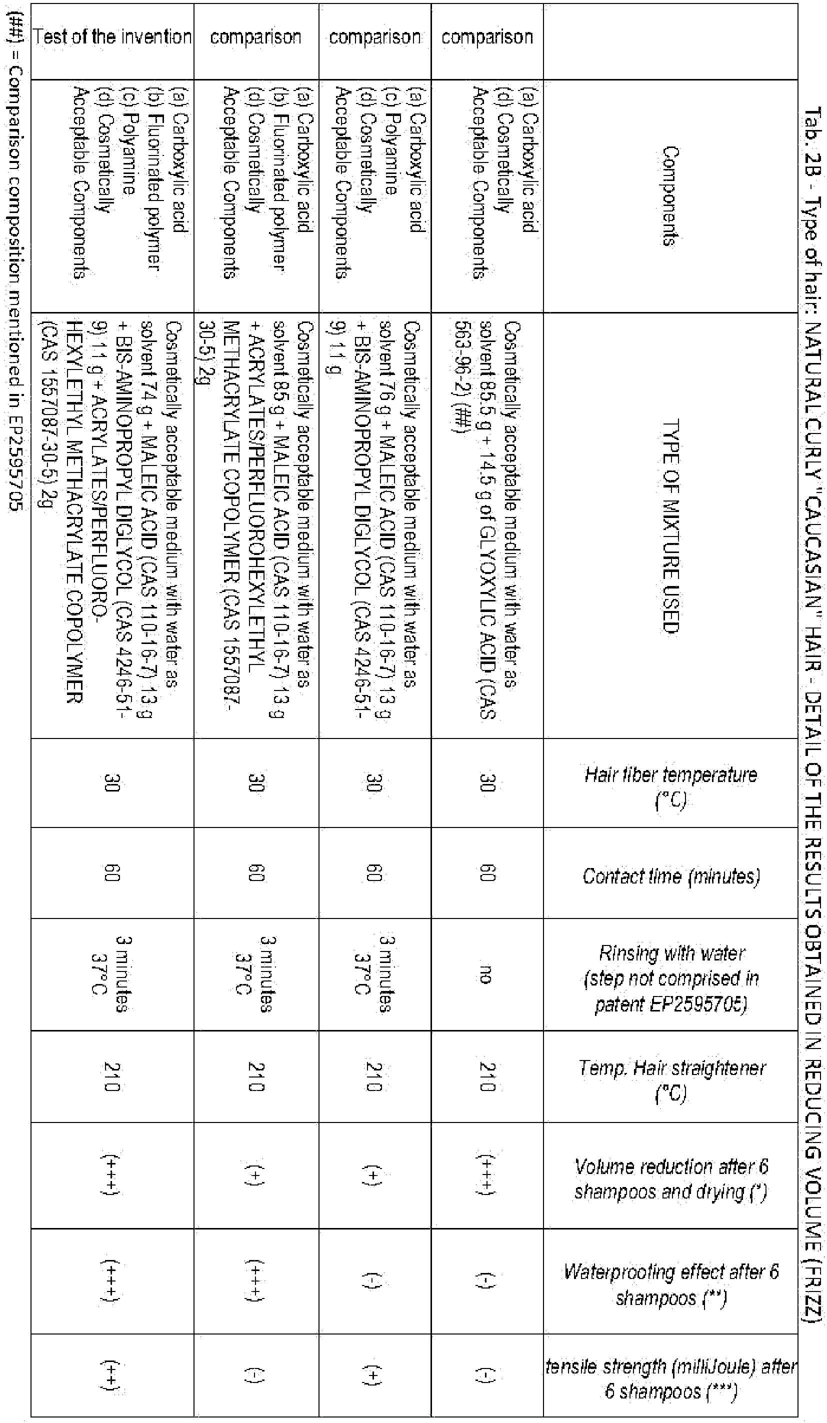

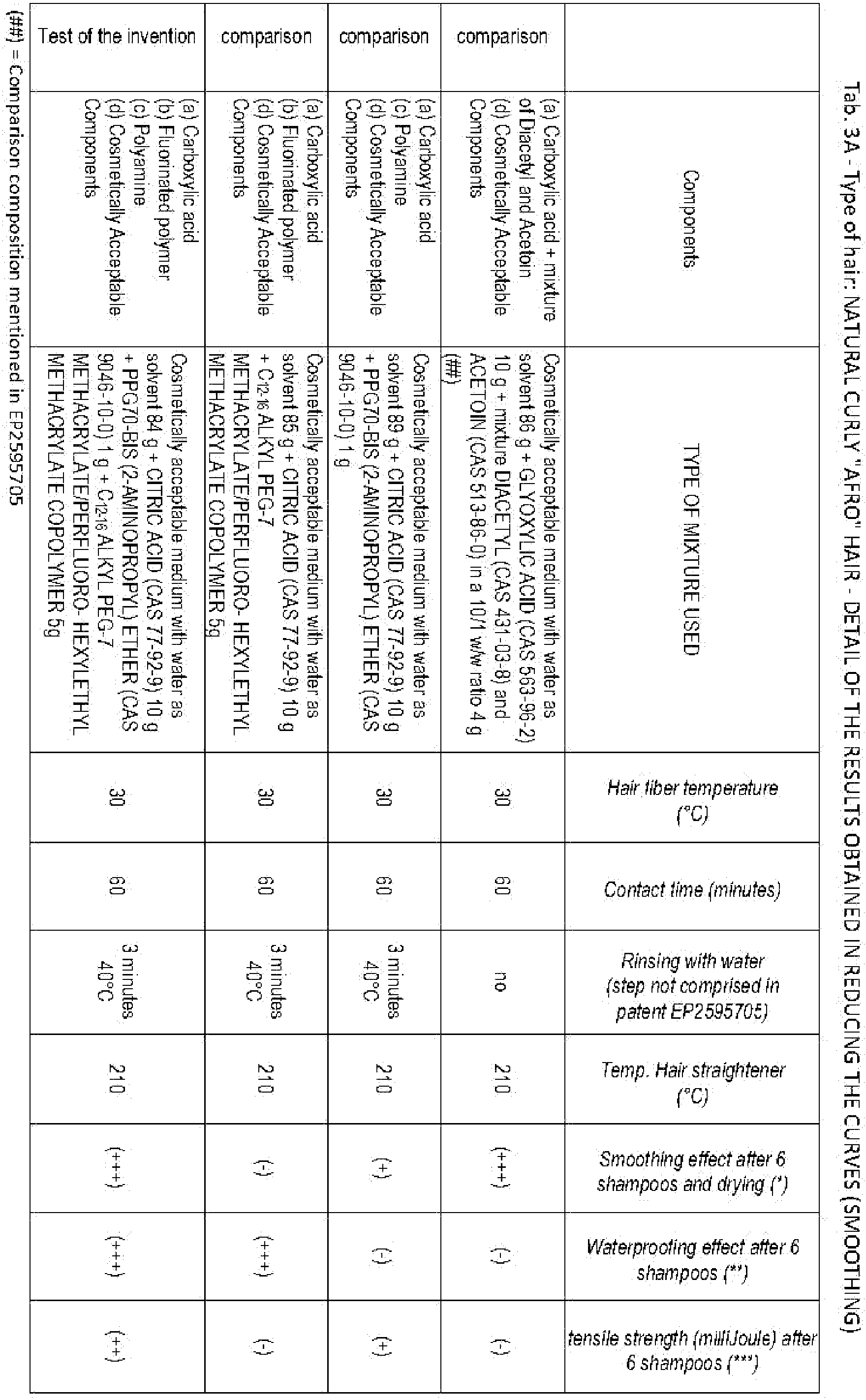

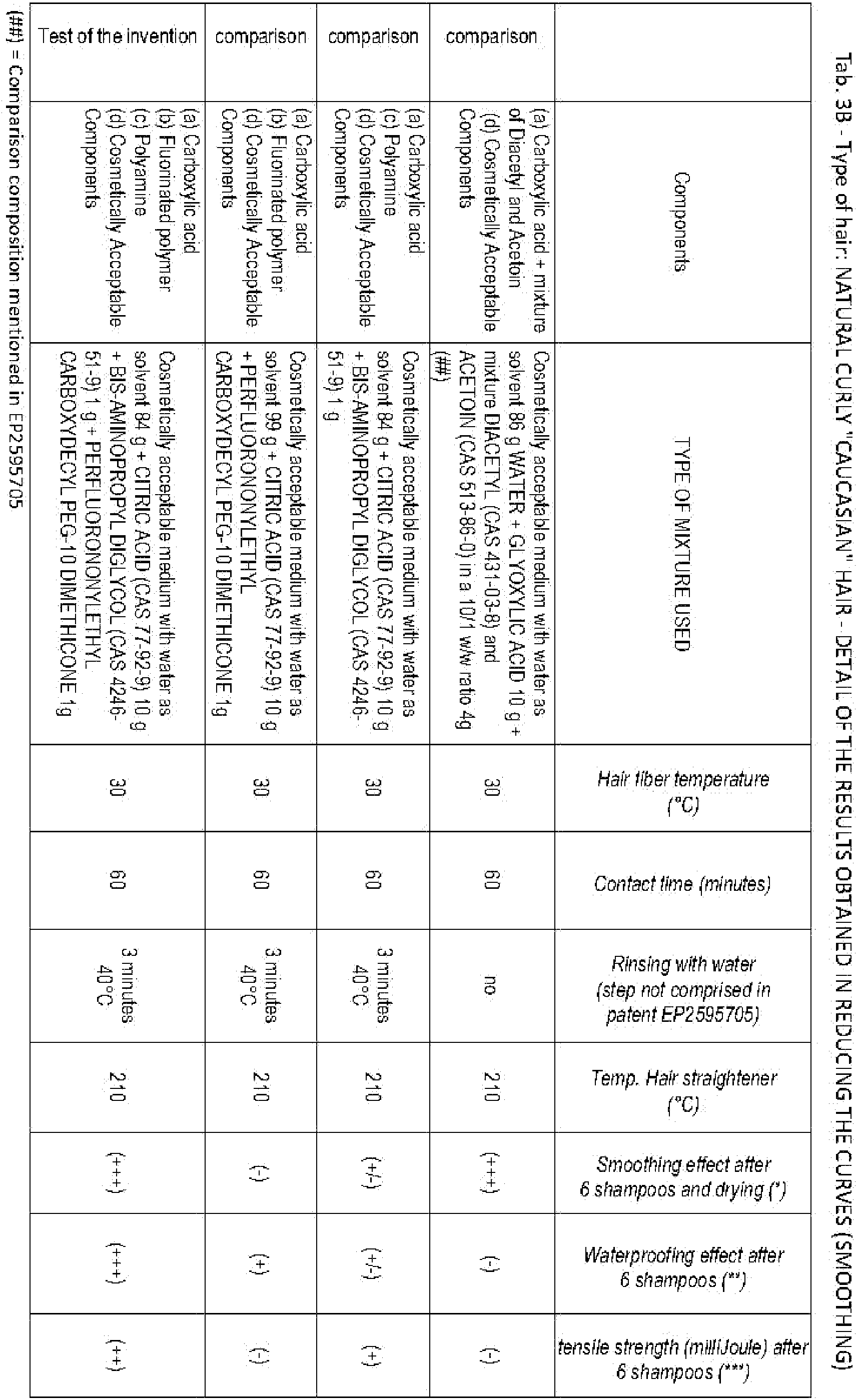

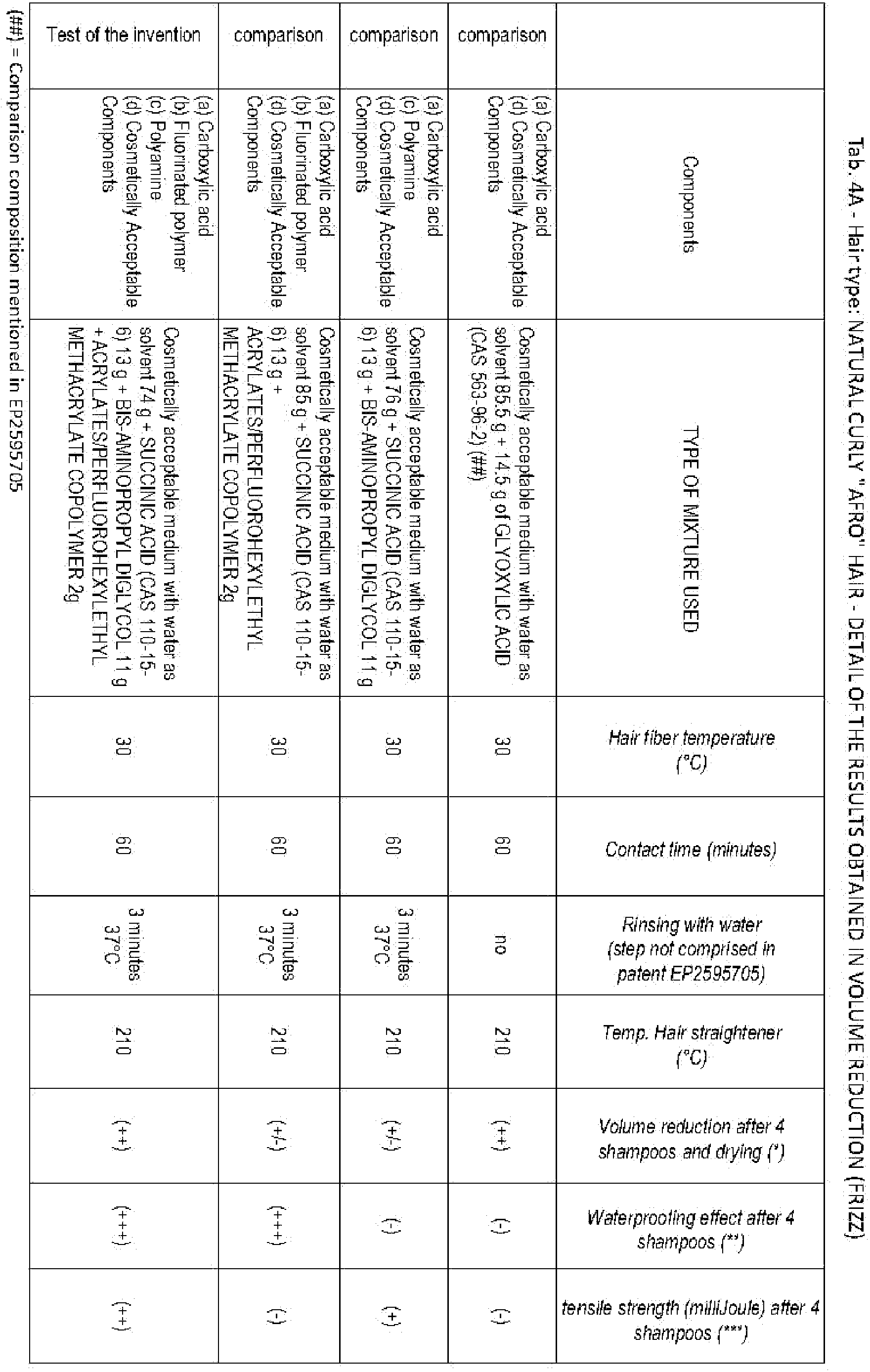

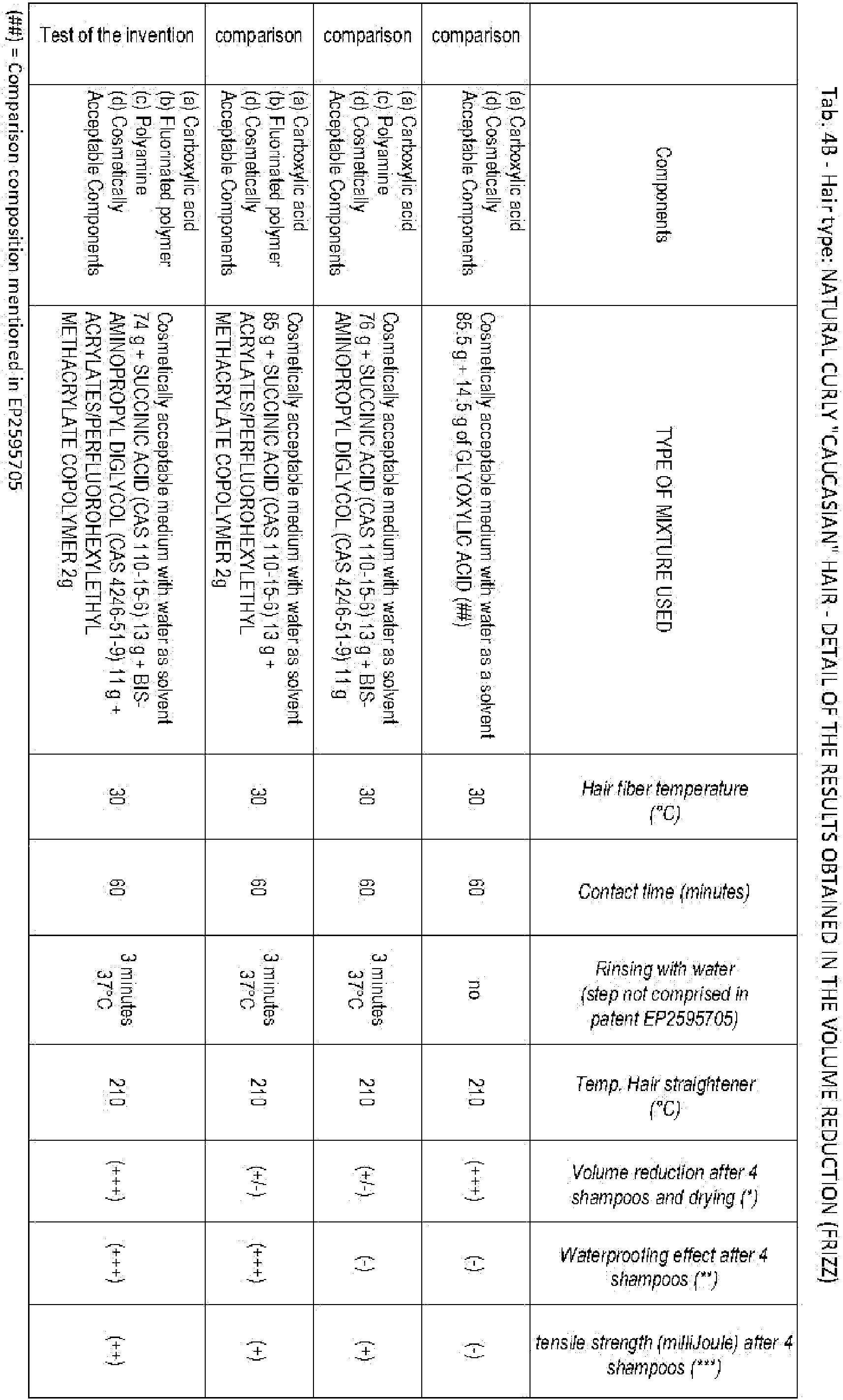

### Example 5:

Similarly, to Examples 1-4, tests were carried out with the compositions of the invention in comparison with the tests shown in the example on pages 26 and 27 of the patent application WO2014072645. The results are shown in Tables 5-8 and there it is highlighted how the performances obtainable with the object of the present invention are much higher than those of the example presented in the patent application WO2014/072645.

With the aim of demonstrating the total difference between the performances obtainable with the object of the present invention with respect to the example reported on pages 26/27 of application WO2014/072645, tests were organized in parallel between the mixtures object of the invention and the relative methods of application and the mixtures and methods of use described in the aforementioned application.

From the tests performed on "afro" and "european" (also known as "caucasian") hair, total absence of performance similar to that obtainable with the object of the invention was found.

In particular, the various mixtures object of the invention show the ability to smooth the curly hair fiber in a way lasting over time (even 6 shampoos), to make it less permeable to water (a property that can also be found after 6 shampoos) and the property to make the hair fiber more resistant to traction (even after 6 shampoos). The same kind of results are obtained in the case of reducing the "frizz" effect.

Conversely, the mixtures described in the example on pages 26 and 27 of application WO2014/072645, applied in the manner mentioned in the same application both on curly hair (with the intention of making it smooth) and on "frizzy" hair (with the intent to reduce its volume) show the inability of the formulations described to give the hair fibers the properties found with the mixtures relating to the present invention. In other words, there is no smoothing effect after 6 shampoos, no kind of waterproofing and no increase in the tensile strength of the fiber.

It would seem that these are substances intended to be used for the modeling (finish) of the hair fibers in the final phase of the cosmetic procedure without modifying the hair fiber with the consequence that the characteristics given to the fibers are lost at the first wash.

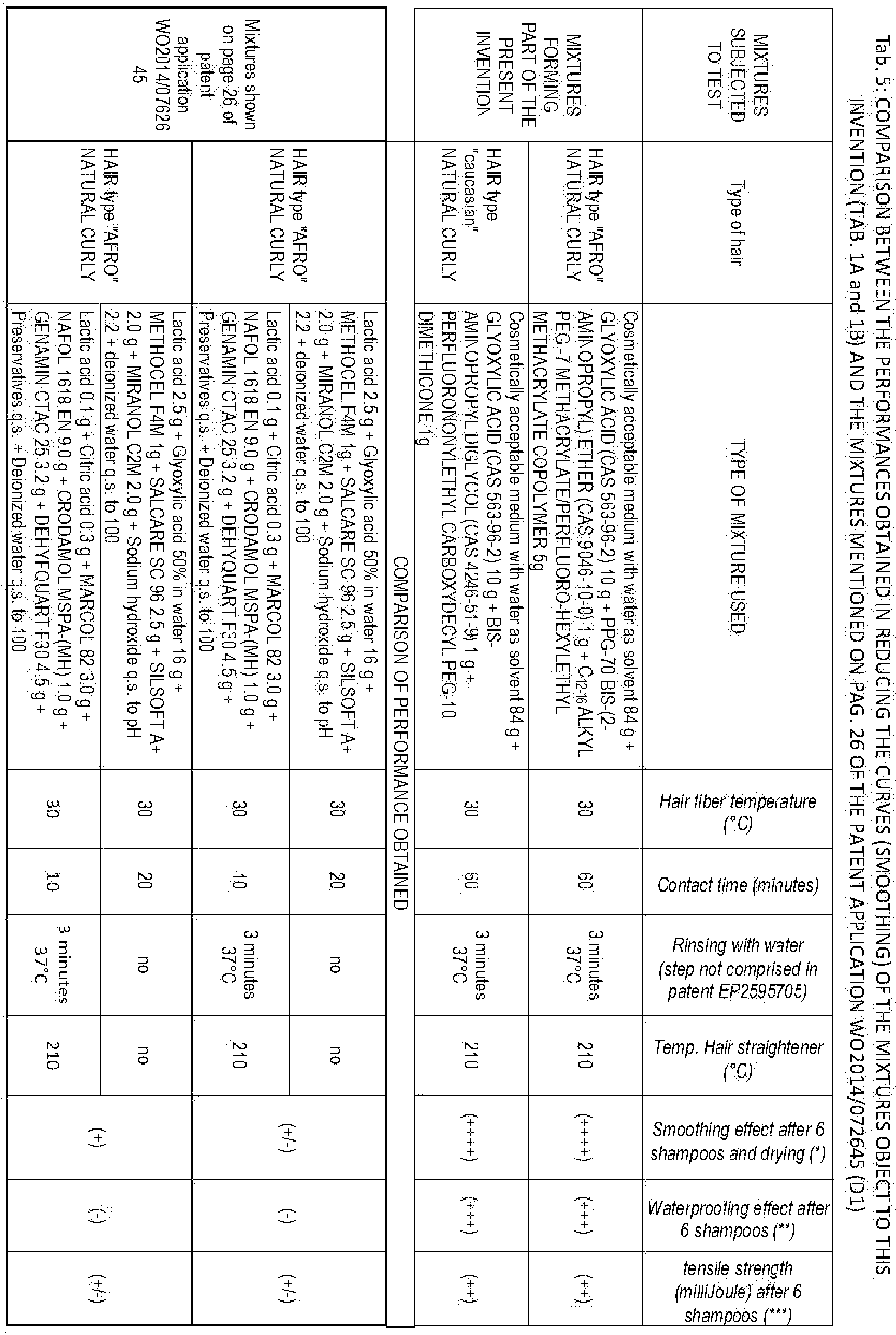

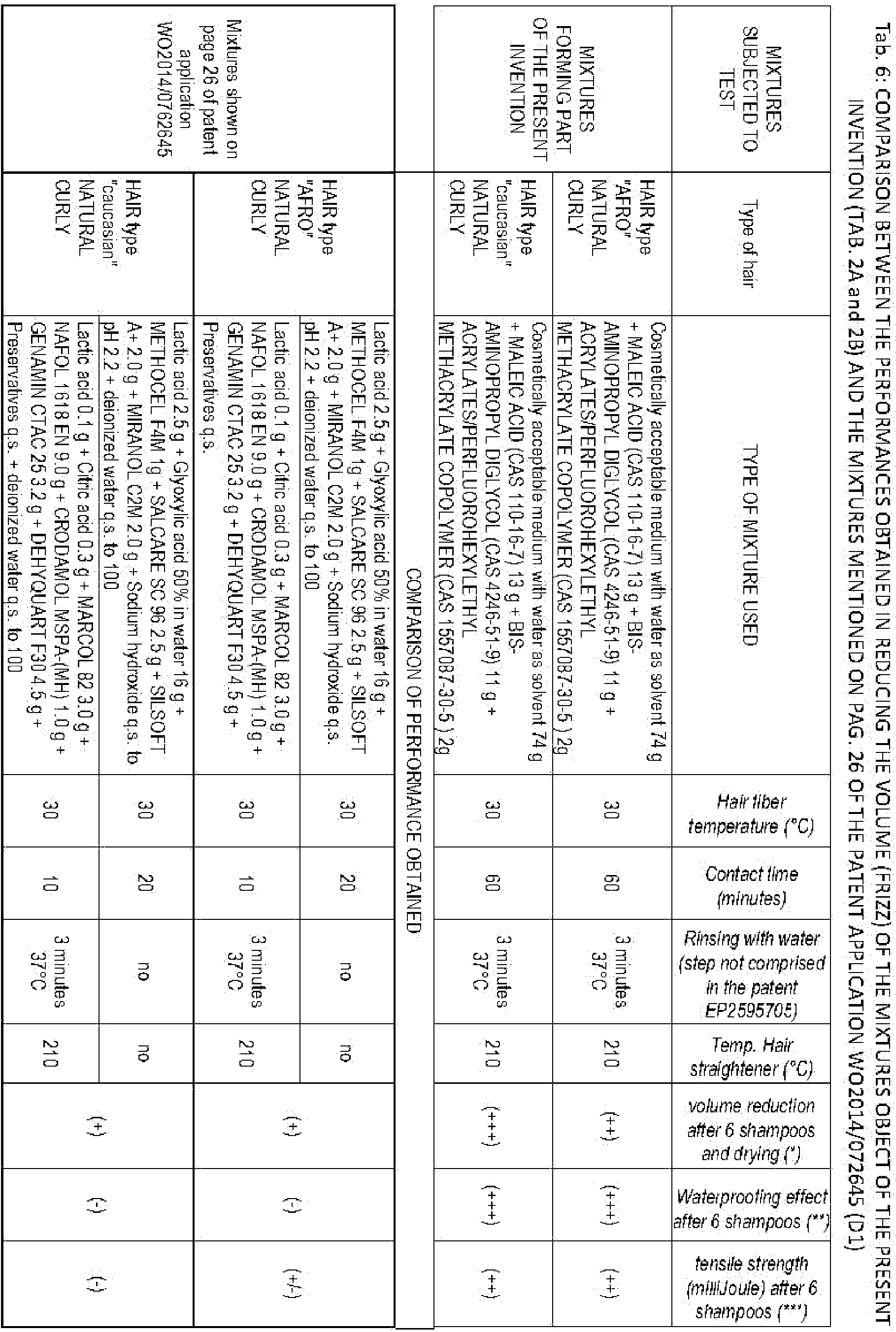

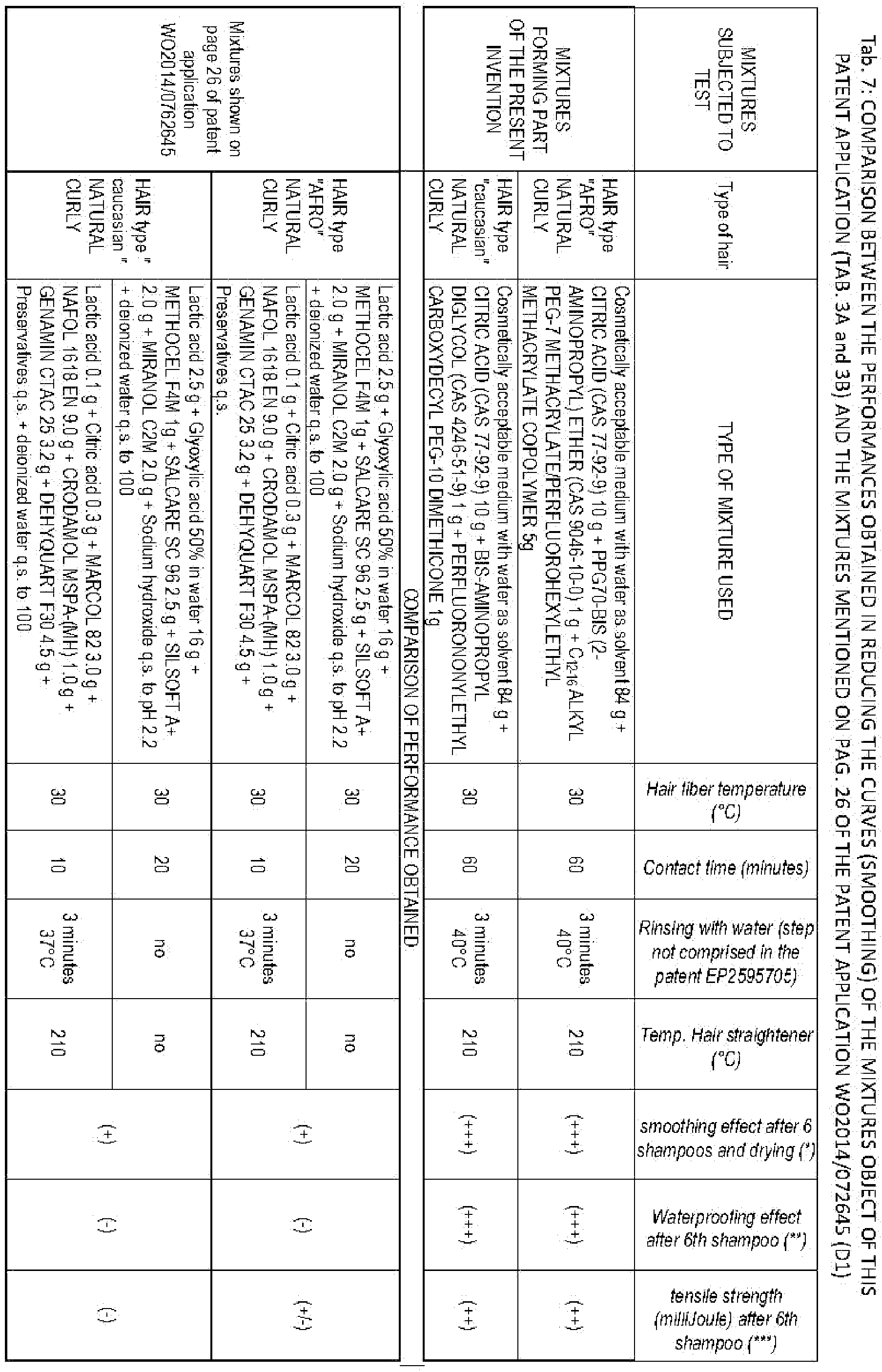

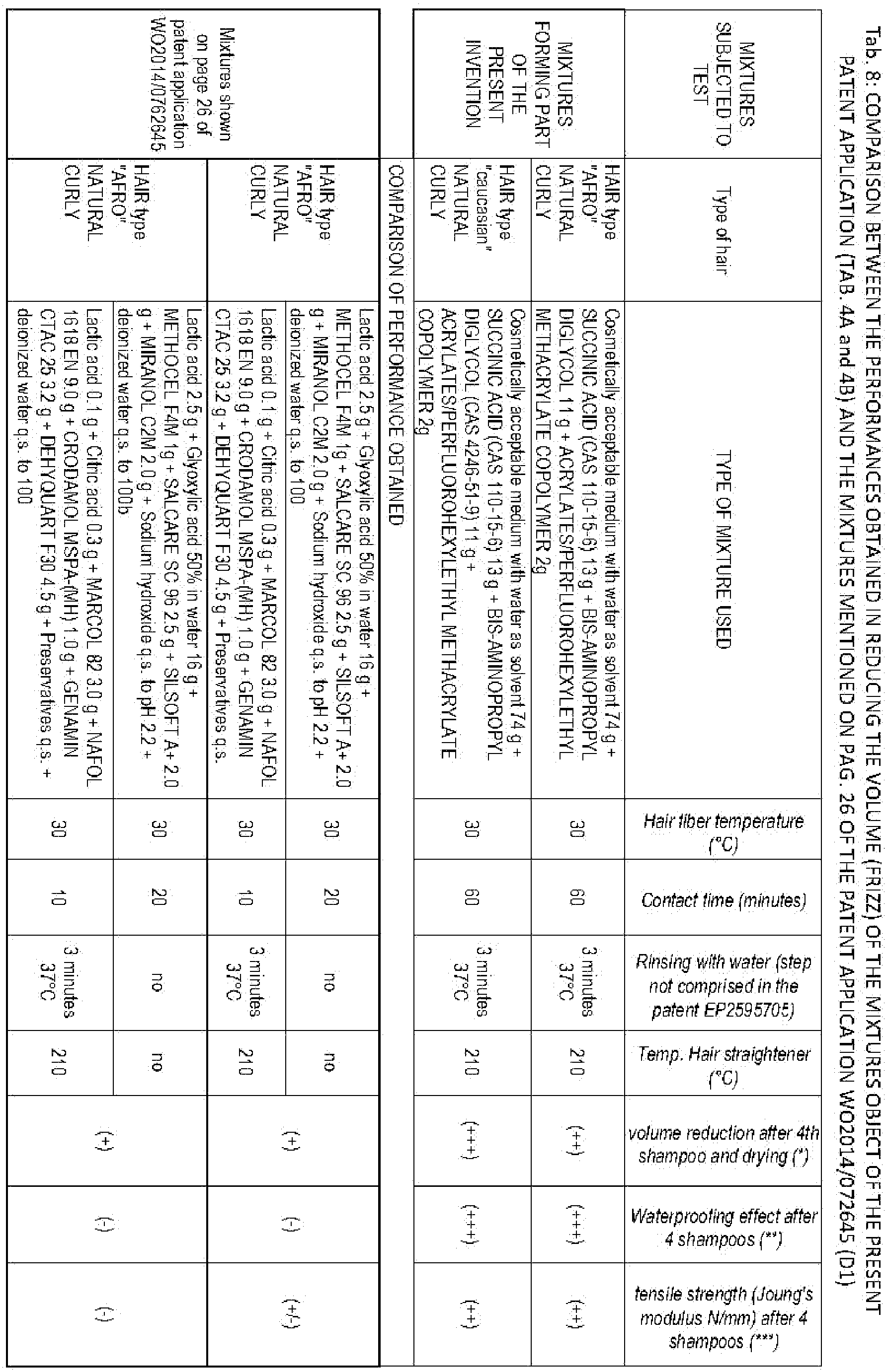

Always referring to the known art, in the patent document WO2014/072645 a copolymer is mentioned which has the commercial name of "SILSOFT A+" whose macromolecule is composed of an alternation of silicone hydrophobic blocks and polyether blocks, as per Scheme 1 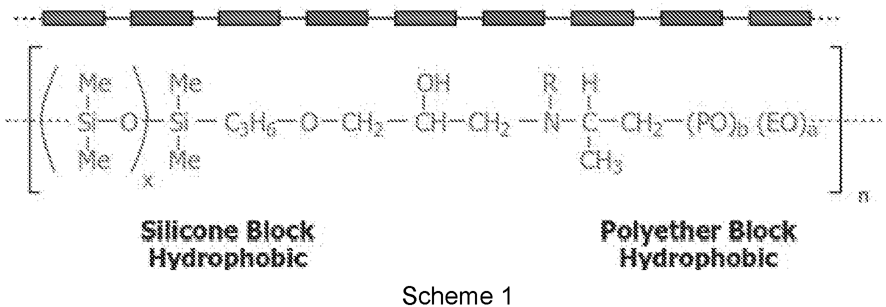 PO = polypropylene oxide; EO = polyethylene oxide

As shown in scheme 1, Silsoft A+ is a very different macromolecule from those of the present invention.

First of all because there are two (2) different types of monomers (an amino-silicone polymer block and a mixed polyethylene + polypropylene glycol polymer block) joined together via the amino silicone nitrogen atom.

Differently from the alkoxylated polyamines of the present invention, in Silsoft A+ there are no primary amino groups (-NH₂) which, as is known, have a much higher reactivity than the tertiary amino groups towards the amino acid residues present in the hair fibers.

The essence of the present invention lies precisely in having found the right combination of three molecules already known, and used individually or in association with other molecules different from those of the invention, for the treatment of keratin fibers.

It has surprisingly been found that the combined use of the three substances that make up the mixture of the invention brings considerable advantages to the keratin fibers through a single application and after rinsing the quantities of substances not absorbed by the capillary fibers (with a consequent increase in the speed of application and greater protection of the physical-mechanical integrity of the hair fibers) compared to the technique of not rinsing the fibers.

## Claims

1. A cosmetic composition for the treatment of keratin fibers comprising:
(a) at least one carboxylic acid having a number of carbon atoms lower than 12 selected from the saturated and unsaturated aliphatic carboxylic acids, their salts and mixtures thereof;
(b) at least one fluorinated polymer selected from perfluoro-acrylic or perfluoro-silicone polymers, and mixtures thereof;
(c) at least one alkoxylated polyamine and mixtures thereof;
(d) at least one component selected from water and a cosmetically acceptable aqueous solution
the composition having a pH between 0.5 and 10.0.

2. The composition according to claim 1 wherein the salts of at least one carboxylic acid are selected from organic and inorganic salts.

3. The composition according to any one of claims 1-2 wherein the carboxylic acid is selected from: adipic acid, citric acid, tartaric acid, malonic acid, glyoxylic acid, dihydroxyacetic acid, fumaric acid, itaconic acid, maleic, lactic acid, malic acid, glutaric acid, succinic acid, glycolic acid, and mixtures thereof; preferably glyoxylic acid, maleic acid, dihydroxyacetic acid, citric acid, succinic acid and mixtures thereof.

4. The composition according to any one of claims 1-3, wherein the carboxylic acid is present in an amount comprised between 0.01 and 30% by weight, preferably between 0.1 and 25% by weight, more preferably between 0,25 and 20% by weight, with respect to the total weight of the composition.

5. The composition according to any one of claims 1-4, wherein the at least one fluorinated polymer is selected from:
- fluorinated polymers having the following general formulas:
• Fluorosilicones insoluble in water with molecular weight between 500 and 200,000 daltons of general formula (W): 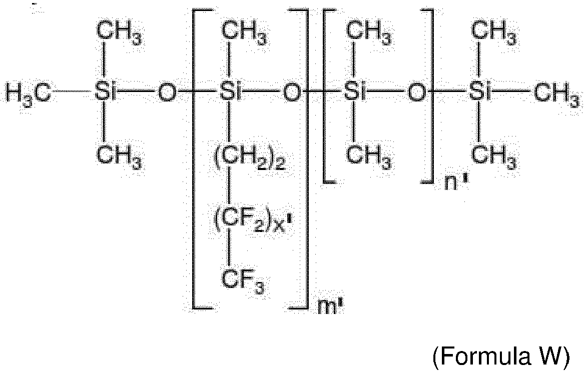 wherein:
x'= integer between 1 and 50,
m'= integer between 10 and 1000,
n'= integer between 10 and 1000.
• Water-soluble fluorosilicones with the following structural formula and with a molecular weight between 500 and 200,000 daltons: 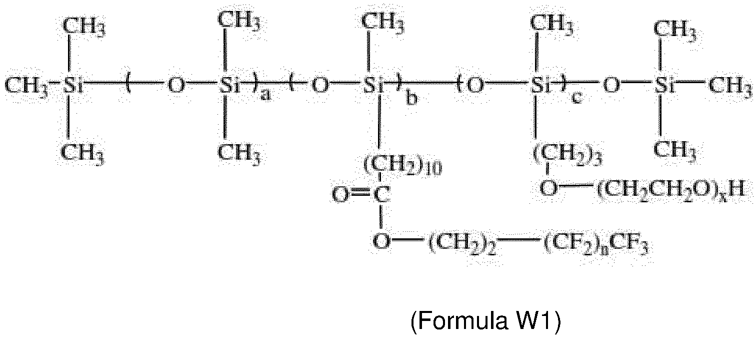 wherein:
a = integer between 10 and 1000,
b = integer between 10 and 1000,
c = integer between 10 and 1000,
n = integer between 1 and 100,
x = integer between 10 and 100
- acrylic and/or methacrylic polymers with non-fluorinated backbone and pendant perfluorinated alkyl groups selected from:
• Diethylaminoethyl methacrylate/hydroxyethyl methacrylate/perfluorohexylethyl methacrylate crosspolymer
• C₆₋₁₄ Perfluoroalkylethyl Acrylate/Hydroxyethyl methacrylate/perfluorohexylethyl methacrylate crosspolymer
• C₁₂₋₁₆ Alkyl PEG-7 Methacrylate/Perfluorohexylethyl Methacrylate copolymer
• Acrylates/Perfluorohexylethyl Methacrylate Copolymer having CAS n. 1557087-30-5
• PEG-10 acrylate/perfluorohexylethyl acrylate copolymer with FDA - USA UNII D76Z87928N Rn 910476-54-9
• Perfluorononyl Dimethicone having CAS n. 259725-95-6,
• Perfluorononylethyl Carboxydecyl PEG-10 Dimethicone having CAS n. 500218-22-4,
• C₁₂₋₁₆ Alkyl PEG-7 Methacrylate/Perfluorohexylethyl Methacrylate copolymer
• C₆₋₁₄ perfluoroalkylethyl acrylate/HEMA copolymer
• Perfluorononylethyl Carboxydecyl PEG-10 Dimethicone and mixtures thereof.

6. The composition according to any one of claims 1-5, wherein the at least one fluorinated polymer is present in an amount between 0.01 and 30% by weight, preferably between 0.1 and 25% by weight, more preferably between 0.25 and 20% by weight, with respect to the total weight of the composition.

7. The composition according to any one of claims 1-6, wherein the at least one alkoxylated polyamine is selected from:
c.1) - Polyetheramines composed of oxyethylene units (C₂) 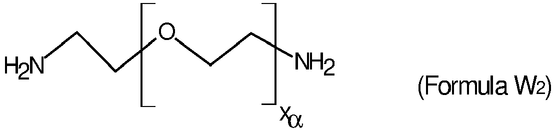 Where Xα is an integer comprised between 1 and 50
c.2) - Polyetheramines composed of oxypropylene units (C₃) 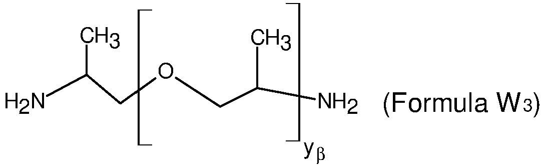 Where yβ is an integer comprised between 1 and 50
c.3) - Polyetheramines composed of mixtures of oxypropylene (C₃) and oxyethylene (C₂) units 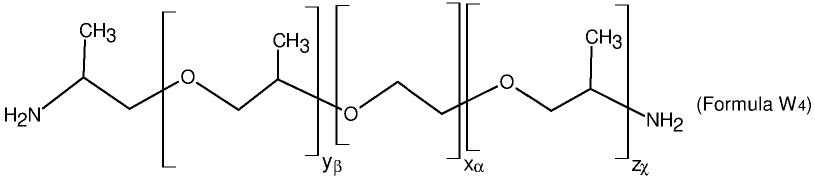 Where xα, yβ and zχ are integers comprised between 1 and 50
c.4) - Polyetheramines composed of tetrahydrofuran units (C₄) 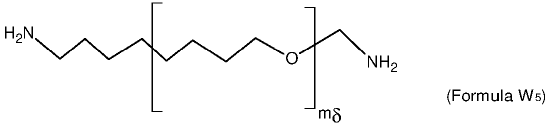 Where mδ is an integer comprised between 1 and 50
c.5) - alkoxylated polyamines of formula (W6):
NH₂(CH₂)ₓ∘(OCH₂CH₂O)_{y°}(CH₂)_{z}∘NH₂ Formula (W6)
where x° and z° are integers between 0 and 3 and y° is an integer between 2 and 100;
• PPG-70 bis-(2-aminopropyl) ether having CAS n. 9046-10-0,
• Bis-Aminopropyl Diglycol with CAS n. 4246-51-9,
and mixtures thereof.

8. The composition according to any one of claims 1-7, wherein the at least one alkoxylated polyamine is present in the composition at a concentration ranging from 0.01 to 30% by weight, preferably from 0.1 to 25% by weight, more preferably between 0.25 and 20% by weight, with respect to the total weight of the composition.

9. The composition according to any one of claims 1-8, wherein the cosmetically acceptable aqueous solution comprises at least one cosmetically acceptable ingredient selected from: anti-caking agent, antifoam, antimicrobial, antioxidant, antistatic, astringent, binder, whitening, buffering, swelling, chelating agent, cleanser, cosmetic dye, denaturing, deodorant, detangling, emollient, emulsifier, emulsion stabilizer, film-forming, flavoring, foaming, gelling, hair conditioning, hair fixative, hair waving or smoothing, humectant, hydrotrope, masking, moisturizing, oxidizing, pearlescent, perfuming, plasticizing, preservative, propellant, reducing, re-greasing, refreshing, soluble smoothing, soothing stabilizer, surfactant, tonic, UV filter, viscosity control substances and mixtures thereof.

10. The composition according to any one of claims 1-9 wherein the at least one cosmetically acceptable ingredient is present in an amount comprised between 0.1 and 90% by weight, preferably between 0.5 and 80% by weight, more preferably between 0.5 and 70% by weight, with respect to the total weight of the composition.

11. The composition according to any one of claims 1-10 which is a ready-to-use mixture and each of the components (a), (b) and (c), has a concentration in the range 0.01-30.0 % of the overall composition.

12. The composition according to any one of claims 1-11 which is formulated in liquid or semi-liquid, fluid or creamy form as solutions, suspensions, lotions, creams.

13. A method for the treatment of keratin fibers, preferably the hair, which comprises the steps of:
- applying on the fibers a composition as defined in claims 1 to 12,
- maintaining said composition in contact with the fibers for 5 to 120 minutes;
- rinsing the fibers and dehumidifying them with hot air;
- smoothing the fibers with a hair straightener at a temperature of about 200 ± 50°C.

## Patentansprüche

1. Kosmetische Zusammensetzung zur Behandlung von Keratinfasern, umfassend:
(a) mindestens eine Carbonsäure mit einer Anzahl von Kohlenstoffatomen von weniger als 12, ausgewählt aus gesättigten und ungesättigten aliphatischen Carbonsäuren, Salzen und Mischungen davon;
(b) mindestens ein Fluorpolymer, ausgewählt aus Perfluoracryl- oder Perfluorsiliziumpolymeren und Mischungen davon;
(c) mindestens ein alkoxyliertes Polyamin und Mischungen davon;
(d) mindestens eine Komponente, ausgewählt aus Wasser und einer kosmetisch akzeptablen wässrigen Lösung
wobei die Zusammensetzung hat einen pH-Wert zwischen 0,5 und 10,0.

2. Zusammensetzung nach Anspruch 1, wobei die Salze mindestens einer Carbonsäure aus organischen und anorganischen Salzen ausgewählt sind.

3. Zusammensetzung nach einem der Ansprüche 1 und 2, wobei die Carbonsäure ausgewählt ist aus: Adipinsäure, Zitronensäure, Weinsäure, Malonsäure, Glyoxylsäure, Dihydroxyessigsäure, Fumarsäure, Itaconsäure, Maleinsäure, Milchsäure, Äpfelsäure, Glutarsäure, Bernsteinsäure, Glykolsäure und Mischungen davon; vorzugsweise Glyoxylsäure, Maleinsäure, Dihydroxyessigsäure, Zitronensäure, Bernsteinsäure und Mischungen davon.

4. Zusammensetzung nach einem der Ansprüche 1 bis 3, wobei die Carbonsäure in einer Menge zwischen 0,01 und 30 Gew.-%, vorzugsweise zwischen 0,1 und 25 Gew.-% und insbesondere zwischen 0,25 und 20 Gew.-%, bezogen auf das Gesamtgewicht der Zusammensetzung, vorhanden ist.

5. Zusammensetzung nach einem der Ansprüche 1 bis 4, wobei der mindestens ein Fluorpolymer ausgewählt ist aus:
- fluorierte Polymere der folgenden allgemeinen Formeln:
• Wasserunlösliche Fluorsilikone mit einer Molekularmasse zwischen 500 und 200.000 Dalton der allgemeinen Formel (W): 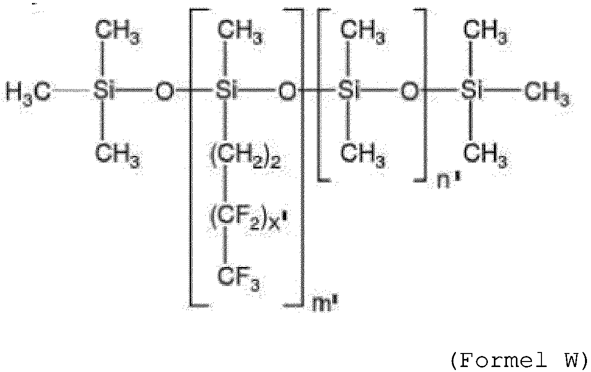 wobei:
x' = Ganzzahl zwischen 1 und 50,
m' = Ganzzahl zwischen 10 und 1000,
n' = Ganzzahl zwischen 10 und 1000.
• Wasserlösliche Fluorsilikone mit folgender Strukturformel und einer Molekularmasse zwischen 500 und 200.000 Dalton: 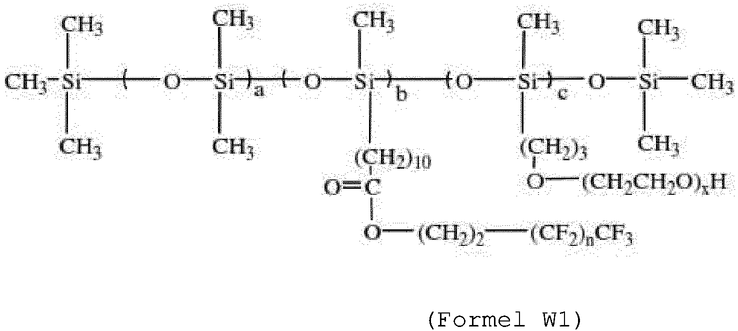 wobei:
a = Ganzzahl zwischen 10 und 1000,
b = Ganzzahl zwischen 10 und 1000,
c = Ganzzahl zwischen 10 und 1000,
n = Ganzzahl zwischen 1 und 100,
x = Ganzzahl zwischen 10 und 100
- Acryl- und/oder Methacrylpolymere mit einem nichtfluorierten Gerüst und seitenständigen perfluorierten Alkylgruppen, ausgewählt aus:
• Vernetztes Polymer aus Diethylaminoethylmethacrylat/Hydroxyethylmethacrylat/P erfluorhexylethylmethacrylat
• Vernetztes Polymer aus C₆₋₁₄ Perfluoralkylethylacrylat/Hydroxyethylmethacrylat/Perf luorhexylethylmethacrylat
• PEG-7 C₁₂₋₁₆ Alkylmethacrylat/Perfluorhexylethylmethacrylat-Copolymer
• Acrylate/Perfluorhexylethylmethacrylat-Copolymer mit der CAS-Nummer: 1557087-30-5
• PEG-10-Acrylat und Perfluorhexylethylacrylat-Copolymer, FDA-zugelassen - USA UNII D76Z87928N Rn 910476-54-9
• Perfluornonyldimethicon (CAS-Nummer: 259725-95-6)
• Perfluorononylethylcarboxydecyl PEG-10 Dimethicon (CAS-Nummer: 500218-22-4)
• Copolymer aus C₁₂₋₁₆-Alkylmethacrylat PEG-7 und Perfluorhexylethylmethacrylat
• Copolymer aus C₆₋₁₄ Perfluoralkylethylacrylat und HEMA
• Perfluorononylethylcarboxydecyl PEG-10 Dimethicon und Mischungen davon.

6. Zusammensetzung nach einem der Ansprüche 1 bis 5, in der mindestens ein Fluorpolymer in einer Menge zwischen 0,01 und 30 Gew.-%, vorzugsweise zwischen 0,1 und 25 Gew.-%, besonders bevorzugt zwischen 0,25 und 20 Gew.-%, bezogen auf das Gesamtgewicht der Zusammensetzung, vorhanden ist.

7. Zusammensetzung nach einem der Ansprüche 1 bis 6, in der mindestens ein alkoxyliertes Polyamin ausgewählt ist aus:
c.1) - Polyetheramine aus Oxyethyleneinheiten (C₂) 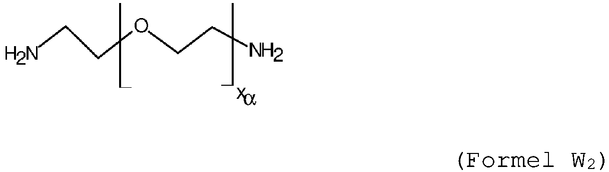 wobei xα ist eine Ganzzahl zwischen 1 und 50
c.2) - Polyetheramine aus Oxypropyleneinheiten (C₃) 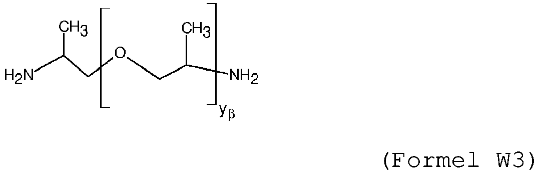 wobei yβ ist eine Ganzzahl zwischen 1 und 50
c.3) - Polyetheramine, bestehend aus Mischungen von Oxypropylen (C3) und Oxyethylen (C2) Einheiten 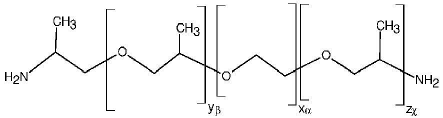 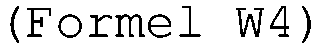 wobei xα, yβ und zχ sind Ganzzahlen zwischen 1 und 50
c.4) - Polyetheramine aus Tetrahydrofuran-Einheiten (C₄) 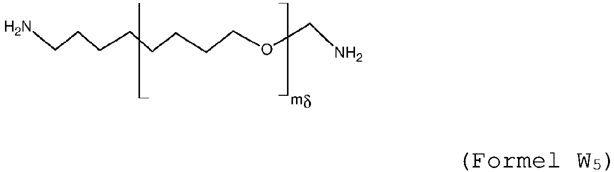 wobei mδ ist eine Ganzzahl zwischen 1 und 50
c.5) - Alkoxylierte Polyamine der Formel (W6):
NH₂(CH₂)ₓ∘(OCH₂CH₂O)_{y}∘(CH₂)_{z}∘NH₂ Formel (W6)
wobei x° und z° sind Ganzzahlen zwischen 0 und 3 und y° ist eine Ganzzahl zwischen 2 und 100;
• PPG-70 Bis-(2-aminopropyl)ether mit der CAS-Nummer 9046-10-0,
• Bis-aminopropyldiglykol mit der CAS-Nummer 4246-51-9 und Mischungen davon.

8. Zusammensetzung nach einem der Ansprüche 1 bis 7, wobei mindestens ein alkoxylierte Polyamin in der Zusammensetzung in einer Konzentration zwischen 0,01 und 30 Gew.-%, vorzugsweise zwischen 0,1 und 25 Gew.-% und besonders bevorzugt zwischen 0,25 und 20 Gew.-%, bezogen auf das Gesamtgewicht der Zusammensetzung, vorhanden ist.

9. Zusammensetzung nach einem der Ansprüche 1 bis 8, wobei die kosmetisch akzeptable wässrige Lösung mindestens einen kosmetisch akzeptablen Bestandteil enthält, ausgewählt aus: Trennmittel, Antischaummittel, antimikrobieller Wirkstoff, Antioxidans, Antistatikum, Adstringens, Bindemittel, Aufheller, Puffer, Füllstoff, Chelatbildner, Reinigungsmittel, kosmetischer Farbstoff, Vergällungsmittel, Deodorant, Entwirrungsmittel, Erweichungsmittel, Emulgator, Emulsionsstabilisator, Filmbildner, Geschmacksstoff, Schaumbildner, Geliermittel, Haarspülung, Haarfestiger, Well- oder Glättmittel, Feuchthaltemittel, Hydrotrop, Maskierungsmittel, Feuchtigkeitsspender, Oxidationsmittel, Perlglanzmittel, Parfüm, Weichmacher, Konservierungsmittel, Treibmittel, Reduktionsmittel, Rückfetter, Erfrischungsmittel, lösliches Glättungsmittel, beruhigender Stabilisator, Tensid, Stärkungsmittel, UV-Filter, Viskositätsregler und Mischungen davon.

10. Zusammensetzung nach einem der Ansprüche 1 bis 9, wobei der mindestens eine kosmetisch akzeptable Inhaltsstoff in einer Menge zwischen 0,1 und 90 Gew.-%, vorzugsweise zwischen 0,5 und 80 Gew.-% und besonders bevorzugt zwischen 0,5 und 70 Gew.-%, bezogen auf das Gesamtgewicht der Zusammensetzung, enthält.

11. Zusammensetzung nach einem der Ansprüche 1 bis 10, die eine gebrauchsfertige Mischung darstellt und in der die Komponenten (a), (b) und (c) jeweils in einer Konzentration zwischen 0,01 und 30,0 % der Gesamtzusammensetzung enthalten sind.

12. Zusammensetzung nach einem der Ansprüche 1 bis 11, formuliert in flüssiger oder halbflüssiger, fluider oder cremiger Form, in Form von Lösungen, Suspensionen, Lotionen oder Cremes.

13. Verfahren zur Behandlung von Keratinfasern, vorzugsweise Haaren, umfassend die folgenden Schritte:
- Auftragen einer Zusammensetzung gemäß den Ansprüchen 1 bis 12 auf die Fasern;
- Halten der Zusammensetzung mit den Fasern für 5 bis 120 Minuten;
- Spülen und Entfeuchten der Fasern mit heißer Luft;
- Glätten der Fasern mit einem Glätteisen bei einer Temperatur von ca. 200 °C ± 50°C.

## Revendications

1. Composition cosmétique pour le traitement des fibres kératiniques, comprenant :
(a) au moins un acide carboxylique ayant un nombre d'atomes de carbone inférieur à 12, choisi parmi les acides carboxyliques aliphatiques saturés et insaturés, leur sels et leur mélanges;
(b) au moins un polymère fluoré choisi parmi des polymères perfluoro-acryliques ou perfluoro-siliconés et leur mélanges;
(c) au moins une polyamine alcoxylée et leur mélanges;
(d) au moins un composant choisi parmi l'eau et une solution aqueuse cosmétiquement acceptable.
la composition ayant un pH compris entre 0,5 et 10,0.

2. Composition selon la revendication 1, dans laquelle les sels d'au moins un acide carboxylique sont choisis parmi des sels organiques et inorganiques.

3. Composition selon l'une quelconque des revendications 1 et 2, dans laquelle l'acide carboxylique est choisi parmi : l'acide adipique, l'acide citrique, l'acide tartrique, l'acide malonique, l'acide glyoxylique, l'acide dihydroxyacétique, l'acide fumarique, l'acide itaconique, l'acide maléique, l'acide lactique, l'acide malique, l'acide glutarique, l'acide succinique, l'acide glycolique et leurs mélanges; de préférence, l'acide glyoxylique, l'acide maléique, l'acide dihydroxyacétique, l'acide citrique, l'acide succinique et leurs mélanges.

4. Composition selon l'une quelconque des revendications 1 à 3, dans laquelle l'acide carboxylique est présent en une quantité comprise entre 0,01 et 30 % en poids, de préférence entre 0,1 et 25 % en poids, plus préférablement entre 0,25 et 20 % en poids, par rapport au poids total de la composition.

5. Composition selon l'une quelconque des revendications 1 à 4, dans laquelle au moins un polymère fluoré est choisi parmi:
- des polymères fluorés de formules générales suivantes:
• Fluorosilicones insolubles dans l'eau de masse moléculaire comprise entre 500 et 200.000 daltons de formule générale (W): 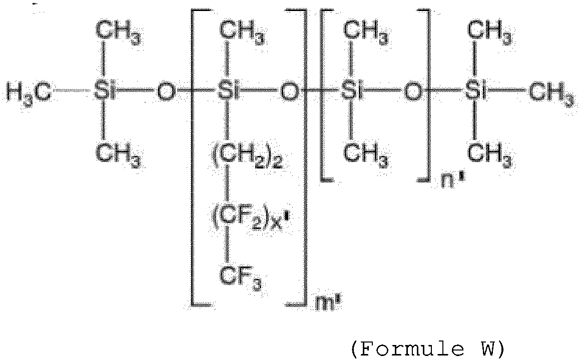 où :
x' = nombre entier compris entre 1 et 50,
m' = nombre entier compris entre 10 et 1000,
n' = nombre entier compris entre 10 et 1000.
• Fluorosilicones hydrosolubles de formule structurelle suivante et de masse moléculaire comprise entre 500 et 200.000 daltons : 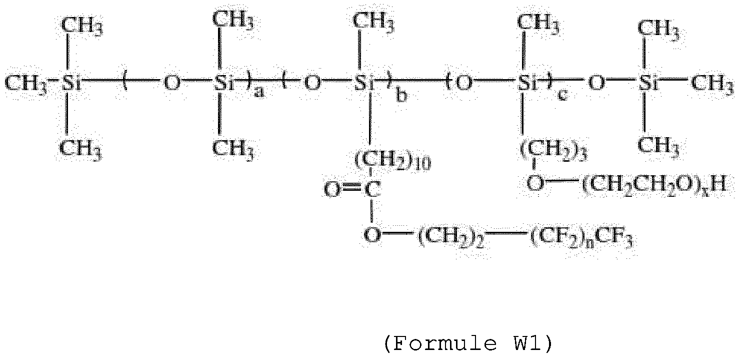 où :
a = nombre entier compris entre 10 et 1000,
b = nombre entier compris entre 10 et 1000,
c = nombre entier compris entre 10 et 1000,
n = nombre entier compris entre 1 et 100,
x = nombre entier compris entre 10 et 100
- des polymères acryliques et/ou méthacryliques à squelette non fluoré et groupes alkyles perfluorés pendants choisis parmi :
• Polymère réticulé de méthacrylate de diéthylaminoéthyle/méthacrylate d'hydroxyéthyle/méthacrylate de perfluorohexyléthyle
• Polymère réticulé d'acrylate de perfluoroalkyléthyle en C₆₋₁₄/méthacrylate d'hydroxyéthyle/méthacrylate de perfluorohexyléthyle
• Copolymère de méthacrylate d'alkyle en C₁₂₋₁₆ PEG-7/méthacrylate de perfluorohexyléthyle
• Copolymère d'acrylates/méthacrylate de perfluorohexyléthyle ayant le numéro CAS : 1557087-30-5
• Copolymère d'acrylate de PEG-10 et d'acrylate de perfluorohexyléthyle, homologué FDA - USA UNII D76Z87928N Rn 910476-54-9
• Perfluorononyldiméthicone (numéro CAS : 259725-95-6)
• Perfluorononyléthylcarboxydécyl PEG-10 diméthicone (numéro CAS : 500218-22-4)
• Copolymère de méthacrylate d'alkyle en C₁₂₋₁₆ PEG-7 et de méthacrylate de perfluorohexyléthyle
• Copolymère d'acrylate de perfluoroalkyléthyle en C₆₋₁₄ et d'HEMA
• Perfluorononyléthylcarboxydécyl PEG-10 diméthicone
et leur mélanges.

6. Composition selon l'une quelconque des revendications 1 à 5, dans laquelle au moins un polymère fluoré est présent en une quantité comprise entre 0,01 et 30 % en poids, de préférence entre 0,1 et 25 % en poids, plus préférablement entre 0,25 et 20 % en poids, par rapport au poids total de la composition.

7. Composition selon l'une quelconque des revendications 1 à 6, dans laquelle au moins une polyamine alcoxylée est choisie parmi :
c.1) - Polyétheramines composées d'unités oxyéthylène (C₂) 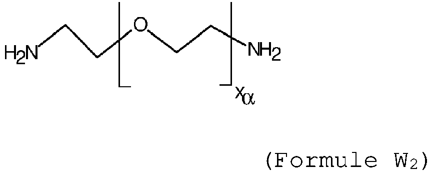 où xα est un nombre entier compris entre 1 et 50
c.2) - Polyétheramines composées d'unités oxypropylène (C₃) 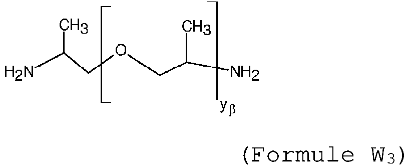 où yβ est un nombre entier compris entre 1 et 50
c.3) - Polyétheramines composées de mélanges d'unités oxypropylène (C₃) et oxyéthylène (C₂) 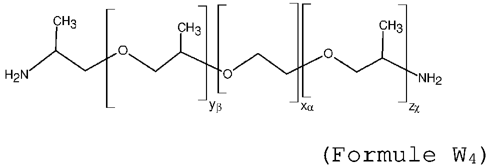 où xα, yβ et zχ sont des nombres entiers compris entre 1 et 50
c.4) - Polyétheramines composées d'unités tétrahydrofurane (C₃) 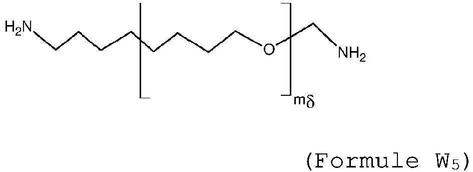 où mδ est un nombre entier compris entre 1 et 50
c.5) - Polyamines alcoxylées de formule (W6) :
NH₂ (CH₂)_{x°}(OCH₂CH₂O)_{y}∘(CH₂)_{z}∘NH₂ Formule (W6)
où x° et z° sont des nombres entiers compris entre 0 et 3 et y° est un nombre entier compris entre 2 et 100;
• Éther bis-(2-aminopropyl) de PPG-70 ayant le numéro CAS 9046-10-0,
• Bis-aminopropyl diglycol ayant le numéro CAS 4246-51-9,
et leurs mélanges.

8. Composition selon l'une quelconque des revendications 1 à 7, dans laquelle la ou les polyamines alcoxylées sont présentes dans la composition à une concentration comprise entre 0,01 et 30% en poids, de préférence entre 0,1 et 25% en poids, et plus préférentiellement entre 0,25 et 20% en poids, par rapport au poids total de la composition.

9. Composition selon l'une quelconque des revendications 1 à 8, dans laquelle la solution aqueuse cosmétiquement acceptable comprend au moins un ingrédient cosmétiquement acceptable choisi parmi: agent antiagglomérant, antimousse, antimicrobien, antioxydant, antistatique, astringent, liant, blanchissant, tampon, gonflant, chélateur, nettoyant, colorant cosmétique, dénaturant, déodorant, démêlant, émollient, émulsifiant, stabilisant d'émulsion, filmogène, aromatisant, moussant, gélifiant, conditionneur capillaire, fixateur capillaire, ondulateur ou lissant, humectant, hydrotrope, masquant, hydratant, oxydant, nacré, parfumant, plastifiant, conservateur, propulseur, réducteur, regraissant, rafraîchissant, lissant soluble, stabilisant apaisant, tensioactif, tonique, filtre UV, substances de contrôle de la viscosité et leur mélanges.

10. Composition selon l'une quelconque des revendications 1 à 9, dans laquelle au moins un ingrédients cosmétiquement acceptable est présent en une quantité comprise entre 0,1 et 90 % en poids, de préférence entre 0,5 et 80 % en poids, et plus préférentiellement entre 0,5 et 70 % en poids, par rapport au poids total de la composition.

11. Composition selon l'une quelconque des revendications 1 à 10, qui est un mélange prêt à l'emploi et dont chacun des composants (a), (b) et (c) présente une concentration comprise entre 0,01 et 30,0% de la composition totale.

12. Composition selon l'une quelconque des revendications 1 à 11, formulée sous forme liquide ou semi-liquide, fluide ou crémeuse, sous forme de solutions, suspensions, lotions, crèmes.

13. Procédé de traitement de fibres kératiniques, de préférence des cheveux, comprenant les étapes suivantes:
- application sur les fibres d'une composition telle que définie dans les revendications 1 à 12;
- maintien de ladite composition en contact avec les fibres pendant 5 à 120 minutes;
- rinçage et déshumidification des fibres à l'air chaud;
- lissage des fibres au fer à lisser à une température d'environ 200 ± 50 °C.
